# EUROPEAN PATENT SPECIFICATION

(11) **EP 0 684 818 B1**
(45) Date of publication and mention of the grant of the patent: **06.05.1999**
(21) Application number: 94908308.3
(22) Date of filing: 10.02.1994
(51) Int. Cl.: A61K 31/165, A61K 31/70

(54) **METHOD OF REVERSING RESISTANCE OF HIV-1 STRAINS TO ZIDOVUDINE**
AUFHEBEN DER RESISTENZ VON HIV-1 LINIEN GEGENÜBER ZIDOVUDINE
PROCEDE D'INVERSION DE LA RESISTANCE DE SOUCHES D'HIV-1 A LA ZIDOVUDINE

(30) Priority: 22.02.1993 US 21286
(43) Date of publication of application: 06.12.1995
(73) Proprietor: JANSSEN PHARMACEUTICA N.V., 2340 Beerse (BE)
(72) Inventor: ANDRIES, Koenraad Jozef Lodewijk Marcel, B-2340 Beerse (BE); STOFFELS, Paulus Aloysius Maria, B-2320 Hoogstraten (BE)
(74) Representative: Wante, Dirk
(86) International application number: EP9400428
(87) International publication number: WO9418963

(56) References cited:
- EP-A- 0 362 162
- EP-A- 0 484 071
- WO-A-92/00952
- CURRENT OPINION IN INFECTIOUS DISEASES vol. 5 , 1992 pages 798 - 805 CH. FLEXNER 'New antiretroviral agents in clinical development'

## Description

The present invention is concerned with a uses for reversing or suppressing the resistance of HIV-1 strains to zidovudine by contacting the virus (or the cells or the patients harbouring such viruses) with non-nucleoside reverse transcriptase inhibitors of the α-APA (α-anilino phenyl acetamide) type.

### Background of the invention

Human beings infected with Human Immunodeficiency Virus Type I (HIV-1) are currently treated with nucleoside reverse transcriptase inhibitors in particular zidovudine (3'-azido-3'-deoxythymidine, AZT) and to a lesser extent, didanosine (dideoxyinosine, ddI), zalcitabine (dideoxycytidine, ddC) or lamivudine (3TC). Effective control of the viral infection is achieved until HIV-1 strains resistant to the medication develop. Generally, the exposure of HIV-1 to zidovudine in patients in the long run results in a progressive decrease in drug sensitivity of the virus. After about 6 months to 1 year the patient becomes clinically resistant, thus rendering the zidovudine therapy inefficaceous. The cause of the decrease in zidovudine sensitivity has been shown to arise from multiple mutations in the HIV-1 reverse transcriptase (RT) gene for example, at codons 41, 67, 70, 215 and 219, resulting in a set of specific amino acid substitutions in the RT protein. The mutations, singly and in various combinations, each contribute to zidovudine resistance. The relatively long period observed before resistant strains appear (6 months or more of zidovudine therapy) is explained by the fact that multiple mutations at specific amino acid residues must accumulate and in particular, that the amino acid substitution Thr²¹⁵ (to Phe or Tyr) requires two nucleoside transitions (ACC to TTC or ACC to TAC) to occur (B. A. Larder, Science, Vol 246. 1155-1158, 1 december 1989).

From some studies it appears that zidovudine resistance can be suppressed to some extent by discontinuing the administration of zidovudine and switching the therapy to another nucleoside reverse transcriptase inhibitor such as didanosine (M. H. St. Clair et al., Science, Vol 253, 1557-1559, 27 september 1991; G. X. McLeod, Antimicrob. Agents Chemother., May 1992, Vol 36 (5), 920-925). In patients where zidovudine therapy failed, it was found that 6 to 12 months from halting zidovudine therapy and starting didanosine therapy, the sensitivity to didanosine decreased whereas the sensitivity to zidovudine unexpectedly increased again. A novel mutation in the RT (Leu⁷⁴ to Val) accounted for the increased resistance to didanosine and the remarkable silencing or suppressing of the effect of the pre-existing zidovudine resistance mutations. Interestingly, the novel mutation when induced in the wild-type HIV-1 (i.e. virus not having the mutant sequences characterizing the zidovudine-resistant HIV-1 strains) did not alter the susceptibility to zidovudine.

Unexpectedly, it has now been found that treatment with a non-nucleoside reverse transcriptase inhibitor of the α-APA type of patients carrying HIV-1 strains with preexisting resistance to zidovudine can reverse said resistance within a short time. This finding indicates that therapy with zidovudine could be maintained, or resumed again earlier, in patients harbouring HIV-1 with pre-existing resistance to zidovudine.

### Description of the invention.

The present invention is concerned with a use of a compound having the formula 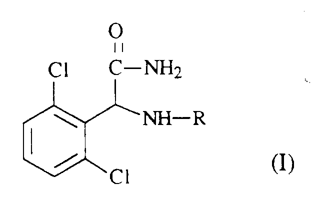 a pharmaceutically acceptable acid addition salt form or a stereochemically isomeric form thereof, wherein R represents 2-nitrophenyl or 2-acetyl-5-methylpheny. for the preparation of a medicament for reversing or suppressing the resistance of HIV-1 strains to zidovudine

The invention further concerns a use of a compound of formula (I) for the preparation of a medicament for treating human cells infected by HIV-1 strains resistant to zidovudine, which comprises administering to said cells an amount of a compound of formula (I), effective in reversing or suppressing the resistance of HIV-1 strains to zidovudine.

Most particularly, the invention concerns the use of a compound of formula (I) for the preparation of a medicament for treating patients suffering from infection by HIV-1 strains resistant to zidovudine.

The compounds of formula (I), their preparation and their anti-retroviral properties against HIV-1 are known from WO 92/00952. The enantiomeric forms of the compounds of formula (I) hereinafter are indicated and/or identified by prefixing their name with the sign of their specific rotation. In particular the laevo-enantiomers [(-)-forms] are preferred in the present methods.

The most preferred compounds are
α-[(2-nitrophenyl)amino]-2,6-dichlorobenzeneacetamide,
α-[(2-acetyl-5-methylphenyl)amino]-2,6-dichlorobenzeneacetamide,
(-)-α-[(2-nitrophenyl)amino]-2,6-dichlorobenzeneacetamide, or
(-)-α-[(2-acetyl-5-methylphenyl)amino]-2,6-dichlorobenzeneacetamide, which is generically known as loviride.

The uses of the present invention derive from the unexpected finding that isolates obtained from patients with pre-existing resistance to zidovudine become susceptible to zidovudine again after a few months of halting zidovudine therapy and switching to a therapy with a compound of formula (I). The therapy with the compounds of formula (I) is most conveniently conducted by administering orally an effective amount of the active ingredient to the patient. In order to maintain effective plasma levels throughout the day, the effective amount is preferably administered as two, three or four subdoses given at suitable intervals (e.g. every 12, 8 or 6 hours). The use of the present invention comprising treatment with a compound of formula (I) may also be performed while the zidovudine treatment is being maintained. The effective amounts of the compounds of formula (I) depend on the nature of the compound and also on the severity of the condition for which the patient is treated (especially the viral load). For example, the daily administered amount of α-[(2-nitrophenyl)amino]-2,6-dichloro-benzeneacetamide can range from 300 to 3000 mg administered as two, three or four subdoses and that of its laevo-enantiomer is about half that amount and thus can range from 150 to 1500 mg administered as two, three or four subdoses. Preferably the daily administered amount of α-[(2-nitrophenyl)amino]-2,6-dichlorobenzeneacetamide consists of three doses of 400 mg each. As a further example, the daily administered amount of α-[(2-acetyl-5-methylphenyl)-amino]-2,6-dichlorobenzeneacetamide can range from 30 to 600 mg administered as two, three or four subdoses and that of its laevo-enantiomer can range from 15 to 300 mg administered as two, three or four subdoses. Preferably, the daily administered amount of (-)-α-[(2-acetyl-5-methylphenyl)amino]-2,6-dichlorobenzene-acetamide consists of three doses of 100 mg each.

### Example

A patient carrying a zidovudine resistant HIV-1 strain with multiple mutations was enrolled in the present study. Zidovudine therapy having lost its effect was discontinued and replaced by treatment with α-[(2-nitrophenyl)amino]-2,6-dichlorobenzeneacetamide (Co. I, 400 mg t.i.d., po) with informed consent of the patient.

### 1. Standard cultures of HIV-1 from lymphocytes or plasma

A standard HIV-1 isolation from fresh lymphocytes and fresh plasma was performed at several time points (two months (60 days) before the start of therapy, 1 month (30 days) and 4 months (120 days) after start of the therapy).

Plasma or lymphocytes from the patient were co-cultured with 3·10⁶ PHA stimulated (phytohemagglutinin, Sigma, St. Louis, Mo., USA) HIV-1 negative donor lymphocytes. Cultures were performed in a 5% CO₂ atmosphere at 37°C. Medium was replaced every 3 to 4 days. When cell death occured, fresh PHA-stimulated donor lymphocytes were added. Every 3 to 4 days, the cultures were monitored for cytopathic effect and for the presence of HIV-1 p24 antigen in the supernatant. Cultures were followed during 5 weeks. A sample was considered positive for virus if the concentration of p24 antigen in the supernatant of the co-culture (determined by a p24 antigen capture test) exceeded 1000 pg per milliliter on a single determination or > 200 pg per milliliter on two consecutive determinations. Supernatants of positive isolates were aliquoted and frozen at -70°C.

### 2. Sensitivity of HIV isolates from the patient to antiviral compounds

An aliquot of a positive isolate was co-cultured with several cultures of PHA-stimulated donor lymphocytes. Serial dilutions of the antiviral compound (α-[(2-nitrophenyl)-amino]-2,6-dichlorobenzeneacetamide (Co. I) or zidovudine) were added to the cultures. Cultures were performed in a 5% CO₂ atmosphere at 37°C. Medium and drugs were replaced every 3 to 4 days. When cell death occured, fresh PHA-stimulated donor lymphocytes were added. The cultures were monitored for cytopathic effect and for the presence of p24 antigen in the supernatant. When p24 levels produced in the controls (without antiviral compound) exceeded 1000 pg per milliliter, a p24 antigen test was performed on all cultures treated with antivirals The concentration of antiviral compound that was needed to inhibit 50% of p24 antigen production of the controls, was calculated (IC50). The sensitivity of the isolates obtained from the patient was determined on several occasions at different times (experiment nos.). The nucleotide sequence of the reverse transcriptase was determined in order to identify mutations selected by exposure of the retrovirus to Co. I. The results obtained thus far are shown in table 1.

**TABLE 1**

| time (days) | experiment no. | IC₅₀ (ng/ml) zidovudine | IC₅₀ (ng/ml) Co.I | mutation |
|---|---|---|---|---|
| - 60 | 1 | - | 41 | I135T, Q207E, |
| | | | | T215F, |
| | 2 | 17 | 70 | K219Q |
| | 3 | >400 | 73 | |
| | 4 | 143 | 54 | |
| 30 | 5 | 123 | 154 | I135T, Q207E, |
| | | | | T215F, |
| | 2 | 3 | 92 | K219Q |
| | 3 | >400 | 223 | |
| | 4 | 17 | 91 | |
| 120 | 5 | <3.2 | >10000 | I135T, Q207E, |
| | | | | T215F, |
| | 6 | <3.2 | >10000 | K219Q, Y181C |
| | 7 | 7 | >10000 | |
| Wild-type | 2 | 1.1 | 13 | Strain IIIB |
| | 3 | 1.0 | 6 | |
| | 4 | 0.8 | 24 | |

The HIV-1 isolate obtained two months before the therapy switch is zidovudine resistant and Co. I sensitive. After one month of therapy no significant changes appear to have occurred, however, after four months, it is found that the HIV-1 isolate has become resistant to Co. I and has acquired again significant sensitivity for zidovudine. A novel mutation (Y181C) appears to have taken place in the RT gene.

## Claims

1. The use of a compound having the formula 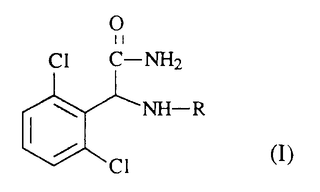 a pharmaceutically acceptable acid addition salt form or a stereochemically isomeric form thereof, wherein R represents 2-nitrophenyl or 2-acetyl-5-methylphenyl for the manufacture of a medicament for reversing or suppressing the resistance of Human Immunodefiency Virus type I (HIV-1) strains to zidovudine (3'-azido-3'-deoxythymidine, AZT).

2. The use of a compound of formula (I) as defined in claim 1 for the manufacture of a medicament for treating human cells infected by HIV-1 strains resistant to zidovudine.

3. The use a compound of formula (I) as defined in claim 1 for the manufacture of a medicament for treating patients suffering from infection by HIV-1 strains resistant to zidovudine of.

4. The use according to claim 3 wherein the compound of formula (I) is administered orally.

5. The use according to claim 1, 2 or 3 wherein the compound is
α-[(2-nitrophenyl)amino]-2,6-dichlorobenzeneacetamide,
α-[(2-acetyl-5-methylphenyl)amino]-2,6-dichlorobenzeneacetamide,
(-)-α-[(2-nitrophenyl)amino]-2,6-dichlorobenzeneacetamide, or
(-)-α-[(2-acetyl-5-methylphenyl)amino]-2,6-dichlorobenzeneacetamide (loviride).

6. The use according to claim 4 wherein the daily administered amount of α-[(2-nitrophenyl)amino]-2,6-dichlorobenzeneacetamide ranges from 300 to 3000 mg administered as two, three or four subdoses or wherein the daily administered amount of its laevo-enantiomer ranges from 150 to 1500 mg administered as two, three or four subdoses.

7. The use according to claim 6 wherein the daily administered amount of α-[(2-nitrophenyl)amino]-2,6-dichlorobenzeneacetamide consists of three doses of 400 mg each.

8. The use according to claim 4 wherein the daily administered amount of α-[(2-acetyl-5-methylphenyl)amino]-2,6-dichlorobenzeneacetamide ranges from 30 to 600 mg administered as two, three or four subdoses or wherein the daily administered amount of its laevo-enantiomer ranges from 15 to 300 mg administered as two, three or four subdoses.

9. The use according to claim 8 wherein the daily administered amount of α-[(2-acetyl-5-methylphenyl)amino]-2,6-dichlorobenzeneacetamide consists of three doses of 100 mg each.

## Patentansprüche

1. Verwendung einer Verbindung der Formel 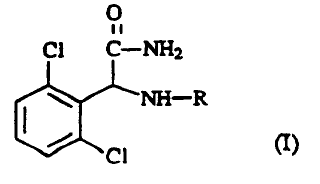 in der R 2-Nitrophenyl oder 2-Acetyl-5-methylphenyl bedeutet, oder einer ihrer pharmazeutisch unbedenklichen Säureadditionssalzformen oder stereochemisch isomeren Formen, zur Herstellung eines Arzneimittels zur Aufhebung oder Unterdrückung der Resistenz von Linien des Human Immunodeficiency Virus Typ I (HIV-1) gegenüber Zidovudin (3'-Azido-3'-desoxythymidin, AZT).

2. Verwendung einer Verbindung der Formel (I) nach Anspruch 1 zur Herstellung eines Arzneimittels für die Behandlung von menschlichen Zellen, die mit zidovudinresistenten HIV-1-Linien infiziert sind.

3. Verwendung einer Verbindung der Formel (I) nach Anspruch 1 zur Herstellung eines Arzneimittels für die Behandlung von Patienten, die mit zidovudin-resistenten HIV-1-Linien infiziert sind.

4. Verwendung nach Anspruch 3, wobei die Verbindung der Formel (I) oral verabreicht wird.

5. Verwendung nach Anspruch 1, 2 oder 3, wobei es sich bei der Verbindung um
α-[(2-Nitrophenyl)amino]-2,6-dichlorbenzolacetamid,
α-[(2-Acetyl-5-methylphenyl)amino]-2,6-dichlorbenzolacetamid,
(-)-α-[(2-Nitrophenyl)amino]-2,6-dichlorbenzolacetamid oder
(-)-α-[(2-Acetyl-5-methylphenyl)amino]-2,6-dichlorbenzolacetamid (Lovirid) handelt.

6. Verwendung nach Anspruch 4, wobei die verabreichte Tagesmenge α-[(2-Nitrophenyl)amino]-2,6-dichlorbenzolacetamid im Bereich von 300 bis 3000 mg liegt und in zwei, drei oder vier Teildosen verabreicht wird, oder wobei die verabreichte Tagesmenge des linksdrehenden Enantiomeren im Bereich von 150 bis 1500 mg liegt und in zwei, drei oder vier Teildosen verabreicht wird.

7. Verwendung nach Anspruch 6, wobei die verabreichte Tagesmenge α-[(2-Nitrophenyl)amino]-2,6-dichlorbenzolacetamid aus drei Dosen zu je 400 mg besteht.

8. Verwendung nach Anspruch 4, wobei die verabreichte Tagesmenge α-[(2-Acetyl-5-methylphenyl)amino]-2,6-dichlorbenzolacetamid im Bereich von 30 bis 600 mg liegt und in zwei, drei oder vier Teildosen verabreicht wird, oder wobei die verabreichte Tagesmenge des linksdrehenden Enantiomeren im Bereich von 15 bis 300 mg liegt und in zwei, drei oder vier Teildosen verabreicht wird.

9. Verwendung nach Anspruch 8, wobei die verabreichte Tagesmenge α-[(2-Acetyl-5-methylphenyl)amino]-2,6-dichlorbenzolacetamid aus drei Dosen zu je 100 mg besteht.

## Revendications

1. Utilisation d'un composé de formule 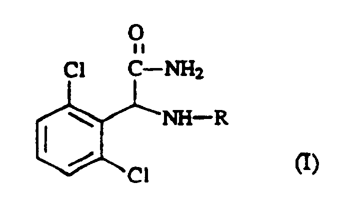 d'une forme en sel d'addition d'acide pharmaceutiquement acceptable de celui-ci ou d'une forme stéréochimiquement isomère de celui-ci, dans laquelle R représente 2-nitrophényle ou 2-acétyl-5-méthylphényle, pour la fabrication d'un médicament destiné à inverser ou à supprimer la résistance de souches du virus de l'immunodéficience humaine de type I (VIH-1) à la zidovudine (3'-azido-3'-désoxythymidine, AZT).

2. Utilisation d'un composé de formule (I) telle que définie dans la revendication 1 pour la fabrication d'un médicament destiné à traiter les cellules humaines infectées par les souches de VIH-1 résistantes à la zidovudine.

3. Utilisation d'un composé de formule (I) telle que définie dans la revendication 1 pour la fabrication d'un médicament destiné à traiter des patients atteints d'une infection par les souches de VIH-1 résistantes à la zidovudine.

4. Utilisation selon la revendication 3, caractérisée en ce que le composé de formule (I) est administré par voie orale.

5. Utilisation selon la revendication 1, 2 ou 3, caractérisée en ce que le composé est
le α-[(2-nitrophényl)amino]-2,6-dichlorobenzèneacétamide,
le α-[(2-acétyl-5-méthylphényl)amino]-2,6-dichlorobenzèneacétamide,
le (-)-α-[(2-nitrophényl)amino]-2,6-dichlorobenzèneacétamide, ou
le (-)-α-[(2-acétyl-5-méthylphényl)amino]-2,6-dichlorobenzèneacétamide (loviride).

6. Utilisation selon la revendication 4, caractérisée en ce que la quantité de α-[(2-nitrophényl)amino]-2,6-dichlorobenzèneacétamide administrée par jour varie de 300 à 3 000 mg administrés en 2, 3 ou 4 sous-doses ou en ce que la quantité de son énantiomère lévo administrée par jour varie de 150 à 1 500 mg administrés en deux, trois ou quatre sous-doses.

7. Utilisation selon la revendication 6, caractérisée en ce que la quantité de α-[(2-nitrophényl)amino]-2,6-dichlorobenzèneacétamide administrée par jour est constituée de trois doses de 400 mg chacune.

8. Utilisation selon la revendication 4, caractérisée en ce que la quantité de α-[(2-acétyl-5-méthylphényl)amino]-2,6-dichlorobenzèneacétamide administrée par jour varie de 30 à 600 mg administrés en deux, trois ou quatre sous-doses ou en ce que la quantité de son énantiomère lévo administrée par jour varie de 15 à 300 mg administrés en deux, trois ou quatre sous-doses.

9. Utilisation selon la revendication 8, caractérisée en ce que la quantité de α-[(2-acétyl-5-méthylphényl)amino]-2,6-dichlorobenzèneacétamide administrée par jour est constituée de trois doses de 100 mg chacune.
